# EUROPÄISCHE PATENTSCHRIFT

(11) **EP 2 825 868 B1**
(45) Veröffentlichungstag und Bekanntmachung des Hinweises auf die Patenterteilung: **04.05.2016**
(21) Anmeldenummer: 13708478.6
(22) Anmeldetag: 12.03.2013
(51) Int. Cl.: G01N 21/84

(54) **TESTSYSTEM UND VERFAHREN ZUR KONTROLLE DER AUSRICHTUNG EINES TESTSTREIFENS**
TEST SYSTEM AND METHOD FOR CONTROLLING THE ALIGNMENT OF A TEST STRIP
SYSTÈME DE TEST ET PROCÉDÉ DE CONTRÔLE DE L'ORIENTATION D'UNE BANDELETTE DE TEST

(30) Priorität: 12.03.2012 EP 12159116
(43) Veröffentlichungstag der Anmeldung: 21.01.2015
(73) Patentinhaber: Roche Diagnostics GmbH, 68305 Mannheim (DE); F.Hoffmann-La Roche AG, 4070 Basel (CH)
(72) Erfinder: LIMBURG, Bernd, 55270 Soergenloch (DE); RUECKERT, Frank, 67071 Ludwigshafen (DE); SCHMIDT, Bernhard, 76865 Insheim (DE)
(74) Vertreter: Pfiz, Thomas
(86) Internationale Anmeldenummer: PCT/EP2013/054963
(87) Internationale Veröffentlichungsnummer: WO 2013/135669

(56) Entgegenhaltungen:
- EP-A2- 0 819 943
- EP-A2- 1 225 442
- WO-A2-2010/105850
- DE-A1- 19 932 846
- US-A1- 2003 169 426

## Beschreibung

Die Erfindung betrifft ein Testsystem für die Analyse einer Körperflüssigkeit, insbesondere zur Blutzuckerbestimmung, mit einem ein analytisches Testfeld aufweisenden Teststreifen, einer photometrischen Messeinheit zur Erfassung von Remissionsmesswerten an dem mit Körperflüssigkeit beaufschlagbaren Testfeld, einem Teststreifenhalter zum Ausrichten des Teststreifens bezüglich der Messeinheit und einer Kontrolleinheit zur Kontrolle der Teststreifenausrichtung, wobei bei korrekter Ausrichtung das Testfeld und bei fehlerhafter Ausrichtung ein das Testfeld einseitig überdeckendes Netzwerk oder eine Trägerfolie des Teststreifens als jeweils einheitlicher Teststreifenbereich in einem optischen Pfad der Messeinheit liegt. Die Erfindung betrifft weiter ein Verfahren zur Kontrolle der Ausrichtung eines Teststreifens in einem solchen Testsystem.

Aus der EP 1 213 579 ist ein Analysesystem mit einer Lagekontrolleinheit bekannt, welche zur Erkennung von vertikalen Lageabweichungen, d.h. Längsbiegungen des Teststreifens eingerichtet ist. Dort wird eine gesonderte Kontroll-Leuchtdiode so angeordnet, dass bei bestimmungsgemäßer Positionierung des Testfelds keine spiegelnd reflektierte Strahlung in den Detektor fällt, während bei einer Aufbiegung des freien Teststreifenendes zunehmend spiegelnd reflektierte Strahlung in den Detektor fällt und das Kontrollsignal ansteigt. Dort sind die Teststreifen an ihrem vorderen (distalen) Ende mit einer Ausnehmung versehen, in die bei korrekter distaler Ausrichtung ein Dorn eingreift. Weiterhin im Stand der Technik bekannt sind Testsysteme, bei denen der Teststreifen eine spezielle Markierung, beispielsweise einen Schwarzbalken aufweist, mit dessen Hilfe die Erkennung der Positionierung des Teststreifens im Messgerät ermöglicht wird.

Aus der DE 19 932 846 A1 ist ein Verfahren zur Erkennung der Fehlpositionierung eines optisch auswertbaren Teststreifens und ein Teststreifen hierfür bekannt.

Ausgehend hiervon liegt der Erfindung die Aufgabe zugrunde, die im Stand der Technik bekannten Systeme und Verfahren weiter zu verbessern und mit möglichst geringem Bau- bzw. Herstellungsaufwand sowohl auf Seiten des Geräts als auch der Verbrauchsmittel die Zuverlässigkeit des Messprozesses zu gewährleisten. Insbesondere sollen fehlerhafte Analysenergebnisse und eine eventuelle Testwiederholung aufgrund von Fehlausrichtungen des Teststreifens vermieden werden.

Zur Lösung dieser Aufgabe wird die in den unabhängigen Patentansprüchen angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, durch Verwendung von mindestens zwei Lichtquellen mit voneinander spektral unterschiedlicher Intensitätsverteilung eine korrekte von möglichen falschen Positionierungen durch das Auftreten grundsätzlich anderer Remissionswerte bestimmen zu können. Dementsprechend wird im Hinblick auf ein Messsystem vorgeschlagen, dass die Messeinheit bei voneinander verschiedenen Wellenlängen jeweils mindestens einen Remissionsmesswert erfasst, und dass die Kontrolleinheit durch einen Vergleich der bei den verschiedenen Wellenlängen erfassten Remissionsmesswerte mit vorgegebenen Referenzwerten eine korrekte oder fehlerhafte Teststreifenausrichtung ermittelt. Dadurch kann auf eine separate Lichtquelle bzw. einen zusätzlichen Detektor für die Lageerkennung verzichtet werden. Auch an dem Teststreifen sind keine zusätzlichen Markierungen erforderlich, da die Abtastung eines einheitlich strukturierten Teststreifenbereichs bei verschiedenen Wellenlängen eine zuverlässige Differenzierung zwischen dem Analysebereich des Testfelds und dem darüber liegenden Spreitnetzwerk am vorderen-Streifenende-und der Trägerfolie am hinteren Ende ermöglicht. Somit lassen sich speziell solche Fehlpositionierungen erkennen, die auf einer Vertauschung von oben und unten bzw. vorne und hinten des Teststreifens bei der Handhabung durch den Benutzer beruhen. Da Fehlmessungen insbesondere im Bereich des Blutzuckermonitorings fatale Folgen nach sich ziehen können, wird durch die Erfindung ein erhöhtes Maß an Gebrauchssicherheit erreicht.

Vorteilhafterweise wird eine wellenlängenunterschiedliche Kontroll- bzw. Messwertgewinnung dadurch erreicht, dass die Messeinheit zwei bei voneinander verschiedenen Wellenlängen emittierende, vorzugsweise durch Leuchtdioden gebildete Lichtquellen aufweist. Die verschiedenen Lichtquellen unterscheiden sich somit voneinander in ihrer spektralen Intensitätsverteilung, während sie gemeinsam auf einen einheitlichen Teststreifenbereich ausgerichtet sind. Der Teststreifenbereich kann dabei je nach Orientierung des Teststreifens durch das Testfeld, das Netzwerk oder die Trägerfolie gebildet sein.

Sowohl hinsichtlich der Positionierkontrolle als auch des eigentlichen Analysevorgangs ist es günstig, wenn die Messeinheit einen ersten Remissionsmesswert in einem ersten Wellenlängenbereich von 600 bis 700 nm, vorzugsweise bei etwa 660 nm und einen zweiten Remissionsmesswert in einem zweiten Wellenlängenbereich von 710 bis 800 nm, vorzugsweise bei etwa 770 nm erfasst.

Eine weitere Verbesserung der Messerfolgs lässt sich dadurch erreichen, dass die Messeinheit drei Lichtquellen aufweist, wobei zwei bei übereinstimmender Wellenlänge emittierende, untereinander gleiche Lichtquellen auf verschiedene Messzonen innerhalb eines einheitlichen Bereichs des Teststreifens ausgerichtet sind.

Eine weitere bauliche Vereinfachung ergibt sich dadurch, dass die Messeinheit einen mit mehreren Lichtquellen zusammenwirkenden Photodetektor zur wellenlängenabhängigen Erfassung von Remissionsmesswerten aufweist. Der Photodetektor bildet somit eine breitbandige Sensoreinheit, die Messwerte bei den verschiedenen Wellenlängen der Lichtquellen erfasst.

Um eine zuverlässige Unterscheidung von der abgewandten Analyseseite des Testfelds zu ermöglichen, ist es vorteilhaft, wenn das Netzwerk so angepasst ist, dass es bei den verschiedenen Wellenlängen der erfassten Remissionsmesswerte einen signifikant unterschiedlichen Remissionsgrad aufweist. Als signifikant wird ein solcher Unterschied angesehen, der außerhalb von Toleranzen bzw. Ungenauigkeiten der Einzelmessungen bei den verschiedenen Wellenlängen liegt.

Zur Verstärkung des wellenlängenabhängigen Remissionsunterschieds ist es von Vorteil, wenn das Netzwerk eine Einfärbung aufweist, die nur bei einer der verwendeten Wellenlängen gut absorbiert. Vorzugsweise wird eine grüne Einfärbung gewählt, um auch den Probenauftrag insbesondere von Blutproben noch deutlich erkennen zu lassen.

Vorteilhafterweise besitzt die Kontrolleinheit einen Speicher zur Speicherung der Referenzwerte und einen elektronischen Prozessor zum Vergleich der Remissionsmesswerte mit den gespeicherten Referenzwerten.

Besonders vorteilhaft für Selbstkontrollen von Patienten sind die Kontrolleinheit und die Messeinheit in einem in der Hand tragbaren Gerät angeordnet, wobei der für den Einmalgebrauch bestimmte Teststreifen in einen an dem Handgerät ausgebildeten Teststreifenhalter einsetzbar ist.

In verfahrensmäßiger Hinsicht wird die eingangs genannte Aufgabe dadurch gelöst, dass ein mit einem analytischen Testfeld versehener Teststreifen in einen Teststreifenhalter eingesetzt und dabei bezüglich einer photometrischen Messeinheit ausgerichtet wird, wobei bei korrekter Ausrichtung das mit Körperflüssigkeit beaufschlagte oder beaufschlagbare Testfeld zur Erfassung von Remissionsmesswerten in einem optischen Pfad der Messeinheit liegt, und dass jeweils mindestens ein Remissionsmesswert bei voneinander verschiedenen Wellenlängen an dem eingesetzten Teststreifen erfasst wird, wobei durch einen Vergleich der bei den verschiedenen Wellenlängen erfassten Remissionsmesswerte mit vorgegebenen Referenzwerten eine korrekte oder fehlerhafte Teststreifenausrichtung ermittelt wird. Damit lassen sich die bereits im Hinblick auf ein Testsystem beschriebenen Vorteile gleichermaßen erreichen.

Eine weiterer vorteilhafter Aspekt der wellenlängenunterschiedlichen Remissionswerterfassung liegt darin, dass durch einen gegenseitigen Vergleich der bei den verschiedenen Wellenlängen erfassten Remissionsmesswerte eine Gebrauchskontrolle des Teststreifens durchführbar ist, wobei bei einer Differenz der Remissionsmesswerte unterhalb eines vorgegebenen Betrages der Teststreifen als benutzbar eingestuft wird.

Vorteilhafterweise werden zur Kontrolle der Ausrichtung oder des Gebrauchszustandes des Teststreifens nur solche Komponenten, insbesondere Lichtquellen der Messeinheit eingesetzt, die auch zur photometrischen Bestimmung eines Analyten in der Körperflüssigkeit verwendet werden.

Eine korrekte Teststreifenausrichtung wird vorteilhafterweise dann festgestellt, wenn alle erfassten Remissionsmesswerte in einem Erwartungsbereich zwischen einem oberen und einem unteren Referenzwert liegen. Umgekehrt kann eine fehlerhafte Teststreifenausrichtung bereits dann festgestellt werden, wenn mindestens ein Remissionsmesswert außerhalb dieses Erwartungsbereichs liegt.

Eine fehlerhafte Ausrichtung und/oder ein Gebrauchszustand des Teststreifens sollte durch eine Fehlermeldung für einen Benutzer ausgegeben werden, wohingegen bei korrekter Ausrichtung und ggf. erfasster Gebrauchsfähigkeit des Teststreifens die Analytbestimmung mittels der photometrischen Messeinheit ohne weiteres erfolgen kann.

Im Folgenden wird die Erfindung anhand eines in der Zeichnung schematisch dargestellten Ausführungsbeispiels näher erläutert. Es zeigen
- Fig. 1: ein Testsystem umfassend ein Blutzuckermessgerät mit eingesetztem Teststreifen in perspektivischer Ansicht;
- Fig. 2: einen Teilschnitt des Testsystems in der Ebene des Teststreifens;
- Fig. 3: einen Schnitt durch die Linie 3-3 in Fig. 2.

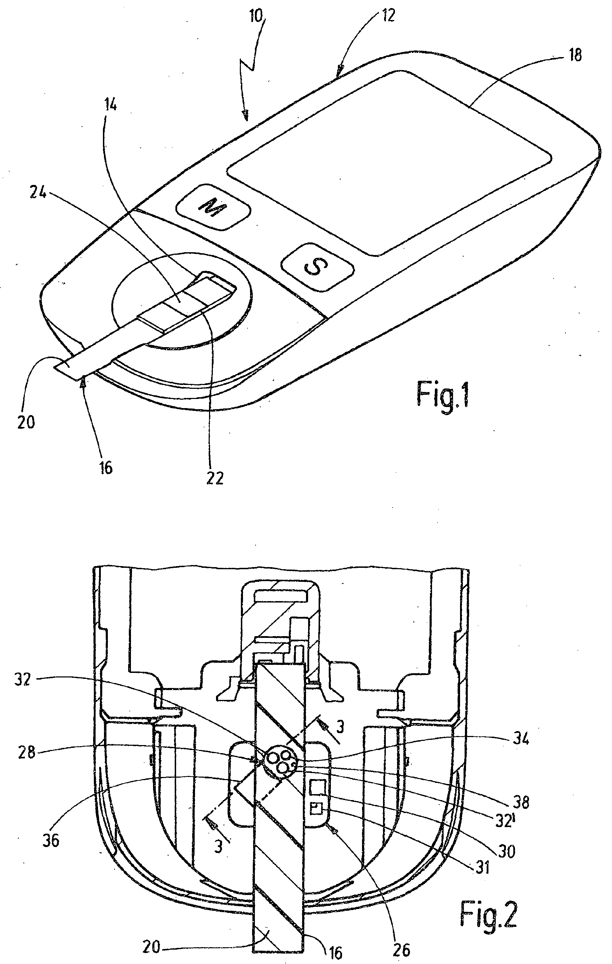

Das in Fig. 1 dargestellte Testsystem 10 umfasst ein tragbares Handgerät 12 und einen in einen geräteseitigen Teststreifenhalter 14 einsetzbaren disposiblen Teststreifen 16 für einen Einmaltest an einer Flüssigprobe, speziell zur Glukosebestimmung in einer Blutprobe, wobei das Messergebnis auf einer Anzeige 18 ausgegeben wird.

Der im Umriss rechteckige Teststreifen 16 weist eine Trägerfolie 20 aus Kunststoff und ein darauf aufgebrachtes, mehrlagiges Testfeld 22 mit einer trockenchemischen Reagenzschicht auf, die an der Oberseite von einem Netzwerk 24 zur flächigen Verteilung einer von oben aufgebrachten Körperflüssigkeit (Blut, ggf. auch Gewebeflüssigkeit oder anderes) überdeckt wird. Das Netzwerk 24 wird durch ein engmaschiges Gewebe gebildet, welches die Probe aufnimmt und kapillaraktiv spreitet bzw. flächig verteilt. Reaktanden innerhalb der Reagenzschicht reagieren irreversibel mit einem Analyten (beispielsweise Glukose) in der aufgebrachten Flüssigkeit. Die so hervorgerufene, optisch detektierbare Veränderung im Remissionsgrad lässt sich auf der Unterseite (der dem Netzwerk 24 gegenüberliegenden Seite) der Reagenzschicht detektieren, wobei ein Messfenster durch eine Ausnehmung oder ein transparentes Material der Trägerfolie 20 vorgesehen ist. Weitere Einzelheiten dazu sind beispielsweise aus der EP 821 233 sowie EP 821 234 entnehmbar, auf die in diesem Zusammenhang explizit Bezug genommen wird.

Die Positionierung des Teststreifens 16 in dem Teststreifenhalter 14 wird von dem Anwender selbst vorgenommen, wobei ggf. auch eingeschränkten Fähigkeiten von Patienten Rechnung zu tragen ist. So sind neben der in Fig. 1 gezeigten lagerichtigen Ausrichtung aufgrund der rechteckigen Streifengeometrie auch Fehlausrichtungen möglich, in denen das Netzwerk 24 nach unten zum Geräteinneren weist oder das proximale Streifenende mit der Ober- oder Unterseite der Trägerfolie 20 voraus in das Gerät 12 eingeschoben wird.

Um solche Fehlausrichtungen zu erkennen, ist in dem Gerät 12 eine Kontrolleinheit 26 in Wirkverbindung mit einer auch für die Analyse bereitgestellten reflektionsphotometrischen Messeinheit 28 vorgesehen, wie es in Fig. 2 symbolisch veranschaulicht ist. Die Kontrolleinheit 26 greift über einen Signalprozessor 30 auf die Remissionsmesswerte der Messeinheit 28 und auf einen Speicher 31 für Referenzwerte zur Lageerkennung zu, wie es weiter unten näher erläutert wird.

Die Messeinheit 28 umfasst drei Leuchtdioden 32, 32',34 und einen beispielsweise als Photodiode ausgebildeten Photodetektor 36. Die bevorzugt untereinander baugleichen Leuchtdioden 32, 32' sind in gleichem seitlichen Abstand zu der Empfängerfläche des Detektors 36. angeordnet und emittieren rotes Licht bei einer ersten Wellenlänge von beispielsweise 660 nm, während die verbleibende Leuchtdiode 34 im Nahinfrarotbereich bei einer zweiten Wellenlänge von beispielsweise 770 nm strahlt. Ein Messloch 38 in der Trägerfolie 20 des Teststreifens 16 ermöglicht eine optische Abtastung des Testfelds 22 von dessen Unterseite her. Möglich ist es auch, dass die beiden gleich beabstandeten Leuchtdioden 32, 32' bei unterschiedlichen Wellenlängen emittieren, oder dass insgesamt nur zwei Leuchtdioden bei voneinander verschiedenen Wellenlängen eingesetzt werden.

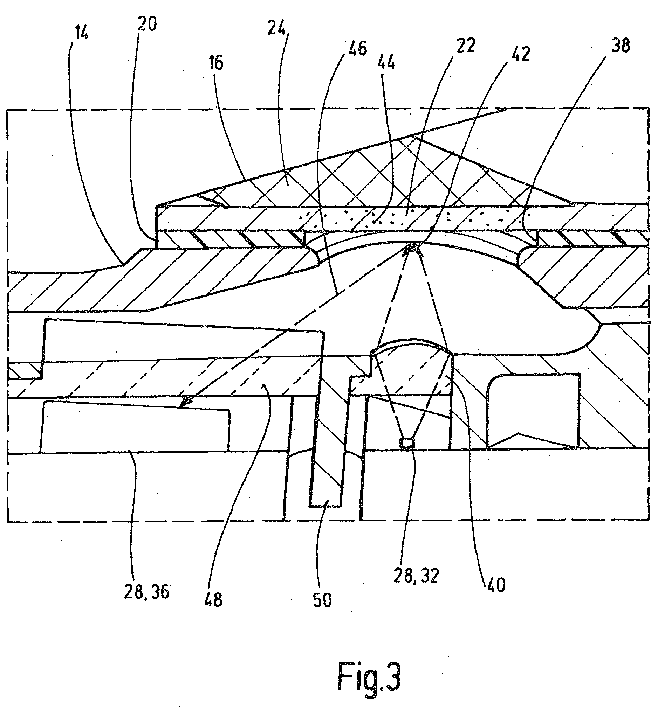

Fig. 3 veranschaulicht den Strahlengang bzw. optischen Pfad der Messeinheit 28 in Bezug auf das Testfeld 22 des Teststreifens 16. Über jeder Leuchtdiode 32, 32', 34 ist eine Sammellinse 40 angeordnet, die das emittierte Licht 42 rückseitig auf das mit Körperflüssigkeit 44 beaufschlagte Testfeld 22 bündelt. Das von dort remittierte (d.h. diffus reflektierte) Licht 46 gelangt über ein transparentes Fenster 48 auf den Detektor 36, während eine gerichtete spiegelnde Reflexion in den Empfangsbereich hinein vermieden wird und ein direktes Lichtübersprechen durch eine Barriere 50 zwischen den Leuchtdioden 32, 32', 34 und dem Detektor 36 verhindert wird. Die erfassten Remissionsmesswerte hängen davon ab, bei welcher Wellenlänge das Licht der Leuchtdioden 32, 32',34 an der bestrahlten Oberfläche stärker absorbiert oder remittiert wird.

Zur Lageerkennung des Teststreifens 16 können mittels des Signalprozessors 30 der Kontrolleinheit 16 die bei den verschiedenen Wellenlängen der Leuchtdioden 32, 32', 34 erfassten Remissionswerte mit in dem Speicher 31 gespeicherten Referenzwerten verglichen werden und als Vergleichsergebnis eine korrekte oder fehlerhafte Teststreifenausrichtung ermittelt werden. Aus der folgenden Tabelle gehen beispielhaft Messwerte des Remissionsgrades hervor, aus denen die korrekte Lage des Teststreifens 16 eindeutig ermittelt werden kann:

| Ausrichtung über der Messeinheit | LED 32 660 nm | LED 34 770 nm | LED 32' 660 nm | Ergebnis |
|---|---|---|---|---|
| Testfeld | 58,5% | 57,9% | 60,7% | richtig |
| Netzwerk | 15,6% | 45,3% | 11,2% | falsch |
| Trägerfolie oben | 91,2% | 86,5% | 81,4% | falsch |
| Trägerfolie unten | 85,2% | 86,9% | 73,5% | falsch |

Der Remissionsgrad gibt das Verhältnis der von der Oberfläche remittierten Leuchtdichte in Messrichtung zu der Leuchtdichte einer Oberfläche in Referenzweiß an. Die Leuchtdioden 32, 32' sind auf verschiedene Messzonen ausgerichtet und können auch aufgrund von Bautoleranzen leicht unterschiedliche Messwerte verursachen.

Eine korrekte Teststreifenausrichtung wird festgestellt, wenn alle erfassten Remissionsmesswerte in einem Erwartungsbereich zwischen 45% und 70% liegen. Diese Werte sind in der Tabelle unterstrichen. Somit lässt sich diejenige Orientierung bzw. Ausrichtung eindeutig als richtig identifizieren, in der das Testfeld 22 nach unten zu der Messeinheit 28 weist. Der Erwartungsbereich kann abhängig von der Herstellungscharge der Teststreifen und gegebenenfalls von deren Alter empirisch bestimmt werden.

Eine fehlerhafte Teststreifenausrichtung wird festgestellt, wenn mindestens ein Remissionsmesswert außerhalb des Erwartungsbereichs von 45% bis 70% liegt. Das Netzwerk 24 absorbiert bei 660 nm stärker als bei 770 nm, so dass hier die kurzwelligen Werte deutlich außerhalb des Erwartungsbereichs liegen. Dieser Effekt kann durch eine geeignete Farbe der Gewebefäden des Netzwerks 24 verstärkt werden. Zweckmäßig wird eine grüne Einfärbung gewählt, so dass der Benutzer auch den Blutauftrag noch gut erkennen kann. Die weiße Trägerfolie 20 wirft das eingestrahlte Licht weitgehend unabhängig von dem Wellenlängenunterschied überwiegend zurück, wodurch in dieser Ausrichtung alle Messwerte oberhalb des Erwartungsbereichs liegen.

Eine weitere Kontrollmöglichkeit durch die wellenlängenabhängige Messung ergibt sich hinsichtlich des Gebrauchszustandes des Teststreifens 16. Wird ein bereits benutzter Teststreifen, selbst mit einer niedrigen Glukosekonzentration der aufgetragenen Blutprobe von weniger als beispielsweise 50 mg/dL, nochmals für eine zweite Messung benutzt, ergibt sich eine Remissionswertdifferenz bei den verschiedenen Wellenlängen aufgrund einer dunkleren Verfärbung des Testfelds 22. Im roten Bereich wird daher weniger Licht remittiert als im Nahinfrarotbereich. Liegt der Betrag der Messwertdifferenz oberhalb einer vorbestimmten Schwelle von beispielsweise 5%, so kann auf eine vorhergehende Benutzung geschlossen werden, während im Bereich darunter der Teststreifen als neu bzw. unbenutzt eingestuft wird.

Im Falle einer Fehlausrichtung wird auf der Anzeige 18 eine Fehlermeldung an den Benutzer ausgegeben, so dass die Fehlausrichtung beseitigt und mit dem gleichen Teststreifen 16 die Analyse dann zuverlässig durchgeführt werden kann. Eine negative Gebrauchskontrolle kann ebenfalls angezeigt werden, um den Teststreifen zu verwerfen.

Die Bestimmung des Analyten kann mit den bereits zuvor beschriebenen Komponenten der Messeinheit 28 erfolgen, wobei neben den beiden bevorzugt baugleichen Mess-LEDs 32, 32' die vom Detektor 36 weiter entfernte dritte LED 34 ein Signal für eine Benetzungskontrolle liefert. Somit sind für die Lageerkennung keine zusätzlichen Bauteile im Gerät oder Markierungen an dem Teststreifen 16 erforderlich.

## Patentansprüche

1. Testsystem für die Analyse einer Körperflüssigkeit, insbesondere zur Blutzuckerbestimmung, mit einem ein analytisches Testfeld (22) aufweisenden Teststreifen (16), einer photometrischen Messeinheit (28) zur Erfassung von Remissionsmesswerten an dem mit Körperflüssigkeit beaufschlagbaren Testfelds (22), einem Teststreifenhalter (14) zum Ausrichten des Teststreifens (16) bezüglich der Messeinheit (28) und einer Kontrolleinheit (26) zur Kontrolle der Teststreifenausrichtung, wobei bei korrekter Ausrichtung das Testfeld (22) in einem optischen Pfad (42,46) der Messeinheit (28) liegt, **dadurch gekennzeichnet, dass** die Messeinheit (28) zwei bei voneinander verschiedenen Wellenlängen emittierende Lichtquellen (32,34) aufweist und jeweils mindestens einen Remissionsmesswert bei den voneinander verschiedenen Wellenlängen erfasst, dass bei fehlerhafter Ausrichtung ein das Testfeld (22) einseitig überdeckendes Netzwerk (24) in einem optischen Pfad (42,46) der Messeinheit (28) liegt, wobei das Netzwerk (24) bei den verschiedenen Wellenlängen der erfassten Remissionsmesswerte einen signifikant unterschiedlichen Remissionsgrad aufweist, und dass die Kontrolleinheit (26) durch einen Vergleich der bei den verschiedenen Wellenlängen erfassten Remissionsmesswerte mit vorgegebenen Referenzwerten eine korrekte oder fehlerhafte Teststreifenausrichtung ermittelt.

2. Testsystem nach Anspruch 1, **dadurch gekennzeichnet, dass** die Messeinheit (28) zwei durch Leuchtdioden gebildete Lichtquellen (32,34) aufweist.

3. Testsystem nach Anspruch 1 oder 2, **dadurch gekennzeichnet, dass** die Messeinheit (28) einen ersten Remissionsmesswert in einem ersten Wellenlängenbereich von 600 bis 700 nm, vorzugsweise bei etwa 660 nm und einen zweiten Remissionsmesswert in einem zweiten Wellenlängenbereich von 710 bis 800 nm, vorzugsweise bei etwa 770 nm erfasst.

4. Testsystem nach einem der Ansprüche 1 bis 3, **dadurch gekennzeichnet, dass** die Messeinheit (28) drei Lichtquellen (32,32',34) aufweist, wobei zwei bei übereinstimmender Wellenlänge emittierende Lichtquellen (32,32') auf verschiedene Messzonen ausgerichtet sind.

5. Testsystem nach einem der Ansprüche 1 bis 4, **dadurch gekennzeichnet, dass** die Messeinheit (28) einen mit mehreren Lichtquellen zusammenwirkenden Photodetektor (36) zur wellenlängenabhängigen Erfassung von Remissionsmesswerten aufweist.

6. Testsystem nach einem der Ansprüche 1 bis 5, **dadurch gekennzeichnet, dass** das Netzwerk (24) eine vorzugsweise grüne Einfärbung aufweist.

7. Testsystem nach einem der Ansprüche 1 bis 6, **dadurch gekennzeichnet, dass** die Kontrolleinheit (26) einen Speicher (31) zur Speicherung der Referenzwerten aufweist.

8. Testsystem nach einem der Ansprüche 1 bis 7, **dadurch gekennzeichnet, dass** die Kontrolleinheit (26) einen elektronischen Prozessor (30) zum Vergleich der Remissionsmesswerte mit den Referenzwerten aufweist.

9. Testsystem nach einem der Ansprüche 1 bis 8, **dadurch gekennzeichnet, dass** die Kontrolleinheit (26) und die Messeinheit (28) in einem Handgerät (12) angeordnet sind, und dass der für den Einmalgebrauch bestimmte Teststreifen (16) in einen an dem Handgerät ausgebildeten Teststreifenhalter (14) einsetzbar ist.

10. Verfahren zur Kontrolle der Ausrichtung eines Teststreifens (16) in einem Testsystem für die Analyse einer Körperflüssigkeit, insbesondere nach einem der vorhergehenden Ansprüche, bei welchem ein mit einem analytischen Testfeld (22) versehener Teststreifen (16) in einen Teststreifenhalter (14) eingesetzt und dabei bezüglich einer photometrischen Messeinheit (28) ausgerichtet wird, wobei bei korrekter Ausrichtung das mit Körperflüssigkeit beaufschlagte oder beaufschlagbare Testfeld (22) zur Erfassung von Remissionsmesswerten in einem optischen Pfad (42,46) der Messeinheit (28) liegt, **dadurch gekennzeichnet, dass** die Messeinheit (28) zwei bei voneinander verschiedenen Wellenlängen emittierende Lichtquellen (32,34) aufweist und jeweils mindestens ein Remissionsmesswert bei den voneinander verschiedenen Wellenlängen an dem eingesetzten Teststreifen (16) erfasst wird, dass bei fehlerhafter Ausrichtung ein das Testfeld (22) einseitig überdeckendes Netzwerk (24) in einem optischen Pfad (42,46) der Messeinheit (28) liegt, wobei das Netzwerk (24) bei den verschiedenen Wellenlängen der erfassten Remissionsmesswerte einen signifikant unterschiedlichen Remissionsgrad aufweist, und dass durch einen Vergleich der bei den verschiedenen Wellenlängen erfassten Remissionsmesswerte mit vorgegebenen Referenzwerten eine korrekte oder fehlerhafte Teststreifenausrichtung ermittelt wird.

11. Verfahren nach Anspruch 10, **dadurch gekennzeichnet, dass** durch einen gegenseitigen Vergleich der bei den verschiedenen Wellenlängen erfassten Remissionsmesswerte eine Gebrauchskontrolle des Teststreifens (16) durchgeführt wird, wobei bei einer Differenz der Remissionsmesswerte unterhalb eines vorgegebenen Betrages der Teststreifen (16) als benutzbar bestimmt wird.

12. Verfahren nach Anspruch 10 oder 11, **dadurch gekennzeichnet, dass** zur Kontrolle der Ausrichtung und/oder zur Gebrauchskontrolle des Teststreifens (16) nur solche Komponenten, insbesondere Lichtquellen (32,32',34) der Messeinheit (28) eingesetzt werden, die auch zur photometrischen Bestimmung eines Analyten in der Körperflüssigkeit verwendet werden.

13. Verfahren nach einem der Ansprüche 10 bis 12, **dadurch gekennzeichnet, dass** eine korrekte Teststreifenausrichtung festgestellt wird, wenn alle erfassten Remissionsmesswerte in einem Erwartungsbereich zwischen einem oberen und einem unteren Referenzwert liegen, und dass eine fehlerhafte Teststreifenausrichtung festgestellt wird, wenn mindestens ein Remissionsmesswert außerhalb des Erwartungsbereichs liegt.

14. Verfahren nach einem der Ansprüche 10 bis 13, **dadurch gekennzeichnet, dass** bei einer fehlerhaften Ausrichtung und/oder einem Gebrauchszustand des Teststreifens (16) eine Fehlermeldung für einen Benutzer ausgegeben wird.

## Claims

1. A test system for analyzing a body fluid, in particular for determining blood sugar levels, comprising a test strip (16) with an analytical test field (22), a photometric measurement unit (28) for acquiring remission measurement values from the test field (22) to which body fluid can be applied, a test strip holder (14) for aligning the test strip (16) with respect to the measurement unit (28) and a monitoring unit (26) for monitoring the test strip alignment, wherein the test field (22) lies in an optical path (42, 46) of the measurement unit (28) in the case of correct alignment **characterized in that** the measurement unit (28) comprises two light sources (32, 34) emitting at different wavelengths and respectively acquires at least one remission measurement value at the differing wavelengths, that a network (24) covering the test field (22) on one side lies in an optical path (42, 46) of the measurement unit (28) in the case of an incorrect alignment, wherein the network (24) has a significantly different remission coefficient at the different wavelengths of the acquired remission measurement values, and **in that** the monitoring unit (26) establishes a correct or incorrect test strip alignment by comparing the remission measurement values acquired at the different wavelengths with predetermined reference values.

2. The test system as claimed in claim 1, **characterized in that** the measurement unit (28) comprises two light sources (32, 34) formed by light-emitting diodes.

3. The test system as claimed in claim 1 or 2, **characterized in that** the measurement unit (28) acquires a first remission measurement value in a first wavelength range from 600 to 700 nm, preferably at approximately 660 nm, and a second remission measurement value in a second wavelength range from 710 to 800 nm, preferably at approximately 770 nm.

4. The test system as claimed in one of claims 1 to 3, **characterized in that** the measurement unit (28) comprises three light sources (32, 32', 34), wherein two light sources (32, 32') emitting with a corresponding wavelength are directed at different measurement zones.

5. The test system as claimed in one of claims 1 to 4, **characterized in that** the measurement unit (28) comprises a photodetector (36) interacting with a plurality of light sources, for wavelength-dependent acquisition of remission measurement values.

6. The test system as claimed in one of claims 1 to 5, **characterized in that** the network (24) has preferably green coloring.

7. The test system as claimed in one of claims 1 to 6, **characterized in that** the monitoring unit (26) comprises a memory (31) for storing the reference values.

8. The test system as claimed in one of claims 1 to 7, **characterized in that** the monitoring unit (26) comprises an electronic processor (30) for comparing the remission measurement values with the reference values.

9. The test system as claimed in one of claims 1 to 8, **characterized in that** the monitoring unit (26) and the measurement unit (28) are arranged in a hand-held instrument (12) and **in that** the test strip (16) designed for single use can be inserted into a test strip holder (14) embodied on the hand-held instrument.

10. A method for monitoring the alignment of a test strip (16) in a test system for analyzing a body fluid, in particular as claimed in one of the preceding claims, in which test system a test strip (16) provided with an analytical test field (22) is inserted into a test strip holder (14) and, in the process, aligned with respect to a photometric measurement unit (28), wherein the test field (22), to which body fluid is or can be applied, for acquiring remission values lies in an optical path (42, 46) of the measurement unit (28) in the case of correct alignment, **characterized in that** the measurement unit (28) comprises two light sources (32, 34) emitting at different wavelengths and in each case at least one remission measurement value is acquired from the inserted test strip (16) at the different wavelengths, that a network (24) covering the test field (22) on one side lies in an optical path (42, 46) of the measurement unit (28) in the case of an incorrect alignment, wherein the network (24) has a significantly different remission coefficient at the different wavelengths of the acquired remission measurement values, and **in that** a correct or incorrect test strip alignment is established by comparing the remission measurement values acquired at the different wavelengths with predetermined reference values.

11. The method as claimed in claim 10, **characterized in that** usage monitoring of the test strip (16) is performed by comparing the remission measurement values acquired at the different wavelengths with one another, wherein the test strip (16) is determined as usable if a difference between the remission measurement values is below a predetermined absolute value.

12. The method as claimed in claim 10 or 11, **characterized in that** only components, in particular light sources (32, 32', 34) of the measurement unit (28), which are also used for photometrically determining an analyte in the body fluid, are used for monitoring the alignment and/or for usage monitoring of the test strip (16).

13. The method as claimed in one of claims 10 to 12, **characterized in that** a correct test strip alignment is determined if all acquired remission measurement values lie in an expectation range between an upper and a lower reference value, and **in that** an incorrect test strip alignment is determined if at least one remission measurement value lies outside of the expectation range.

14. The method as claimed in one of claims 10 to 13, **characterized in that** an error message is output for a user in the case of an incorrect alignment and/or a usage state of the test strip (16).

## Revendications

1. Système de test pour l'analyse d'un liquide organique, en particulier pour la détermination de la glycémie, comprenant une bandelette de test (16) qui présente une zone de test analytique (22), une unité de mesure photométrique (28) pour la détection de valeurs de mesure de réflectance sur la zone de test (22) recevant le liquide organique, un support de bandelette de test (14) servant à orienter la bandelette de test (16) par rapport à l'unité de mesure (28) et une unité de contrôle (26) servant à contrôler l'orientation de la bandelette de test, sachant que, lorsque l'orientation est correcte, la zone de test (22) est située dans un trajet optique (42, 46) de l'unité de mesure (28), **caractérisé en ce que** l'unité de mesure (28) comporte deux sources de lumière (32, 34) émettant à des longueurs d'onde différentes les unes des autres et détecte au moins une valeur de mesure de réflectance à chacune desdites longueurs d'onde différentes les unes des autres, **en ce que**, lorsque l'orientation est incorrecte, un réseau (24) recouvrant la zone de test (22) sur un côté est situé dans un trajet optique (42, 46) de l'unité de mesure (28), ledit réseau (24) présentant un degré de réflectance significativement différent aux différentes longueurs d'onde des valeurs de mesure de réflectance détectées, et **en ce que** l'unité de contrôle (26) détermine une orientation correcte ou incorrecte de la bandelette de test en comparant les valeurs de mesure de réflectance détectées aux différentes longueurs d'onde avec des valeurs de référence prédéterminées.

2. Système de test selon la revendication 1, **caractérisé en ce que** l'unité de mesure (28) comporte deux sources de lumière (32, 34) formées par des diodes électroluminescentes.

3. Système de test selon la revendication 1 ou 2, **caractérisé en ce que** l'unité de mesure (28) détecte une première valeur de mesure de réflectance dans une première gamme de longueurs d'onde de 600 à 700 nm, de préférence à environ 660 nm, et une deuxième valeur de mesure de réflectance dans une deuxième gamme de longueurs d'onde de 710 à 800 nm, de préférence à environ 770 nm.

4. Système de test selon l'une des revendications 1 à 3, **caractérisé en ce que** l'unité de mesure (28) comporte trois sources de lumière (32, 32', 34), deux sources de lumière (32, 32') émettant à des longueurs d'onde concordantes étant orientées sur des zones de mesure différentes.

5. Système de test selon l'une des revendications 1 à 4, **caractérisé en ce que** l'unité de mesure (28) comporte un photodétecteur (36) coopérant avec plusieurs sources de lumière pour la détection dépendante de la longueur d'onde de valeurs de mesure de réflectance.

6. Système de test selon l'une des revendications 1 à 5, **caractérisé en ce que** le réseau (24) présente une coloration de préférence verte.

7. Système de test selon l'une des revendications 1 à 6, **caractérisé en ce que** l'unité de contrôle (26) comporte une mémoire (31) pour mémoriser les valeurs de référence.

8. Système de test selon l'une des revendications 1 à 7, **caractérisé en ce que** l'unité de contrôle (26) comporte un processeur électronique (30) pour la comparaison des valeurs de mesure de réflectance avec les valeurs de référence.

9. Système de test selon l'une des revendications 1 à 8, **caractérisé en ce que** l'unité de contrôle (26) et l'unité de mesure (28) sont disposées dans un appareil à main (12), et **en ce que** la bandelette de test (16) destinée à un usage unique peut être insérée dans un support de bandelette de test (14) formé sur l'appareil à main (12).

10. Procédé de contrôle de l'orientation d'une bandelette de test (16) dans un système de test pour l'analyse d'un liquide organique, en particulier selon l'une des revendications précédentes, dans lequel une bandelette de test (16) pourvue d'une zone de test analytique (22) est insérée dans un support de bandelette de test (14) et est orientée par rapport à une unité de mesure photométrique (28), sachant que, lorsque l'orientation est correcte, la zone de test (22) recevant ou pouvant recevoir le liquide organique pour la détection de valeurs de mesure de réflectance est située dans un trajet optique (42, 46) de l'unité de mesure (28), **caractérisé en ce que** l'unité de mesure (28) comporte deux sources de lumière (32, 34) émettant à des longueurs d'onde différentes les unes des autres et qu'au moins une valeur de mesure de réflectance est détectée à chacune desdites longueurs d'onde différentes les unes des autres sur la bandelette de test (16) insérée, **en ce que**, lorsque l'orientation est incorrecte, un réseau (24) recouvrant la zone de test (22) sur un côté est situé dans un trajet optique (42, 46) de l'unité de mesure (28), ledit réseau (24) présentant un degré de réflectance significativement différent aux différentes longueurs d'onde des valeurs de mesure de réflectance détectées, et **en ce qu'**une orientation correcte ou incorrecte de la bandelette de test est déterminée en comparant les valeurs de mesure de réflectance détectées aux différentes longueurs d'onde avec des valeurs de référence prédéterminées.

11. Procédé selon la revendication 10, **caractérisé en ce qu**'un contrôle de l'usage de la bandelette de test (16) est effectué par une comparaison mutuelle des valeurs de mesure de réflectance détectées aux différentes longueurs d'onde, la bandelette de test (16) étant déterminée comme étant utilisable lorsque la différence des valeurs de mesure de réflectance est inférieure à une valeur prédéterminée.

12. Procédé selon la revendication 10 ou 11, **caractérisé en ce que** pour le contrôle de l'orientation et/ou pour le contrôle de l'usage de la bandelette de test (16) ne sont utilisés que les composants, en particulier les sources de lumière (32, 32', 34) de l'unité de mesure (28), qui sont utilisés aussi pour détermination photométrique d'un analyte dans le liquide organique.

13. Procédé selon l'une des revendications 10 à 12, **caractérisé en ce qu**'une orientation correcte de la bandelette de test est constatée lorsque toutes les valeurs de mesure de réflectance détectées se situent dans une plage attendue comprise entre une valeur de référence haute et une valeur de référence basse, et en ce qu'une orientation incorrecte de la bandelette de test est constatée lorsqu'au moins une valeur de mesure de réflectance se situe en dehors de la plage attendue.

14. Procédé selon l'une des revendications 10 à 13, **caractérisé en ce qu**'en cas d'orientation incorrecte et/ou d'état usé de la bandelette de test (16), un message d'erreur est délivré pour un utilisateur.
